# EUROPEAN PATENT SPECIFICATION

(11) **EP 2 648 672 B1**
(45) Date of publication and mention of the grant of the patent: **01.05.2019**
(21) Application number: 11776424.1
(22) Date of filing: 26.10.2011
(51) Int. Cl.: A61H 1/00, A61M 21/02

(54) **DEVICE FOR MULTISENSORY STIMULATION**
VORRICHTUNG FÜR MULTISENSORISCHE STIMULATION
DISPOSITIF DE STIMULATION MULTISENSORIELLE

(30) Priority: 09.12.2010 IT MI20100367 U; 16.06.2011 IT MI20111082
(43) Date of publication of application: 16.10.2013
(73) Proprietor: Habiche, Leila Benedicte, 20145 Milano (IT)
(72) Inventor: Habiche, Leila Benedicte, 20145 Milano (IT)
(74) Representative: Hautier IP
(86) International application number: PCT/EP2011/068794
(87) International publication number: WO 2012/076248

(56) References cited:
- WO-A1-2006/015335
- WO-A1-2009/018422
- WO-A1-2011/029069
- DE-U1-202005 016 015
- JP-A- 2005 058 404
- US-A- 5 574 339
- US-B1- 6 494 850

## Description

### Technical Field

The present invention relates to a device for multisensory stimulation of the type specified in the preamble of the first claim. Similar devices are described in patent documents WO-A-2009/018422, WO-A-2006/015335 and US-B-6494850.

US-A-5574339 discloses a device according to the preamble of claim 1.

### Background art

As is known, important studies, such as those of professors Wade Marshall and Wilder Penfield, have highlighted the correlations among the several sensory organs and the parts of the brain, demonstrating the importance of correct sensory stimulation on cerebral activity.

These studies are based on the fact that cerebral tissue, differently from the other organic tissues of our body, has an organization, from neurons down to its ultramicroscopic structures, that is determined to a large extent by the stimuli of the environment in which it finds itself in the course of life.

In detail, while the basic structural organization is predetermined by genetic factors, the growth and subsequent outcome and interneuronal recovery are determined by environmental stimuli, which accordingly are fundamental in brain growth and in reinforcing interneural connections. Multisensory stimulation is therefore fundamental in many brain functions, such as for example information transfer, learning capacity, neuron plasticity, reorganization of sensory maps, which occurs for example following an amputation of a limb.

In particular, sensory stimulation is applied to individuals with physical disabilities, in which improvements have been observed in the areas of motivations, concentration and coordination; to people with cognitive disabilities, where it is used to improve relaxation and to provide stimuli that would be otherwise impossible to achieve; to people with psychiatric problems, where it is used to provide the ideal situation for the development of therapeutic relationships; and finally to individuals affected by acute and chronic pain, on whom it has a "rehumanizing" effect due to the deep relaxation and to the perception of a better quality of life.

Multisensory activity is currently performed only in specific centers provided with suitably equipped rooms known as "Snoezelen Rooms".

Such rooms are designed by seeking an optimization of one or more environmental elements (screens for external light, colors of walls, ceilings and floors, textures of surfaces); visual elements (disk image projectors, light effects); tactile elements (vibrating musical armchairs); musical elements (specifically selected lounge music); and olfactory elements (aromas in the environment).

The above cited background art suffers some important drawbacks.

The "Snoezelen Room", due to the complexity of the parameters, is in fact particularly expensive both as far as design is concerned and during use.

This problem causes this type of therapy to be particularly expensive and therefore not accessible to everyone.

Another problem linked to the above cited high costs is that, despite the countless benefits of multisensory stimulation, the diffusion of these therapies is extremely limited.

A further problem is constituted by adjustment, which due to the large number of parameters is complex even for a skilled technician.

A consequent problem is the complexity of the use of these rooms, which requires the presence of a technician.

### Disclosure of the invention

In this situation, the aim of the present invention is to provide a device for multisensory stimulation that can obviate substantially the cited drawbacks.

Within this aim, an object of the invention is to provide a device for multisensory stimulation that is simple to use and has a low cost.

Another object of the invention is to provide a device that makes this therapy executable without the presence of a technician.

This aim and these objects are achieved by a device for multisensory stimulation as claimed in the appended claim 1.

Preferred embodiments are highlighted in the dependent claims.

### Brief description of the drawings

The characteristics and advantages of the invention will become better apparent from the detailed description of a preferred embodiment thereof, with reference to the accompanying drawings, wherein:
Figure 1 is a view of a device helpful for understanding the invention. This device comprises a movement mechanism different from the one of the invention.
Figure 2 is a top view of a component of the device of figure 1;
Figure 3 is a side view of the device of Figure 1;
Figure 4 is a side view of the device for sensory stimulation according to a second embodiment.

### Ways of carrying out the invention

With reference to the figures, the device for multisensory stimulation according to the invention is generally designated by the reference numeral 1.

The device comprises a support 2, which is adapted to support the user in the correct position; a base structure 3, which is connected to the ground or to a resting surface and is adapted to keep the support 2 in a raised position with respect to the ground; a movement mechanism 4, which is adapted to create a relative motion of the support with respect to the base structure 3; a stimulation apparatus 5, which is accommodated at least partially in the base structure 3 and is adapted to stimulate at least part of the senses of the user; and an electronic card 6, which is adapted to control the movement mechanism 4 and the stimulation apparatus 5.

In particular, the base structure 3 is provided with an inner cavity that is adapted to accommodate inside it, at least partially, the movement mechanism 4, the stimulation apparatus 5 and the electronic card 6. Preferably, the structure 3 accommodates inside it all of the stimulation apparatus 5 and of the mechanism 4. Finally, the structure has suitable openings which allow the stimuli produced by the above cited apparatus to reach the user and provide a motion transfer connection between the mechanism 4 and the support 2.

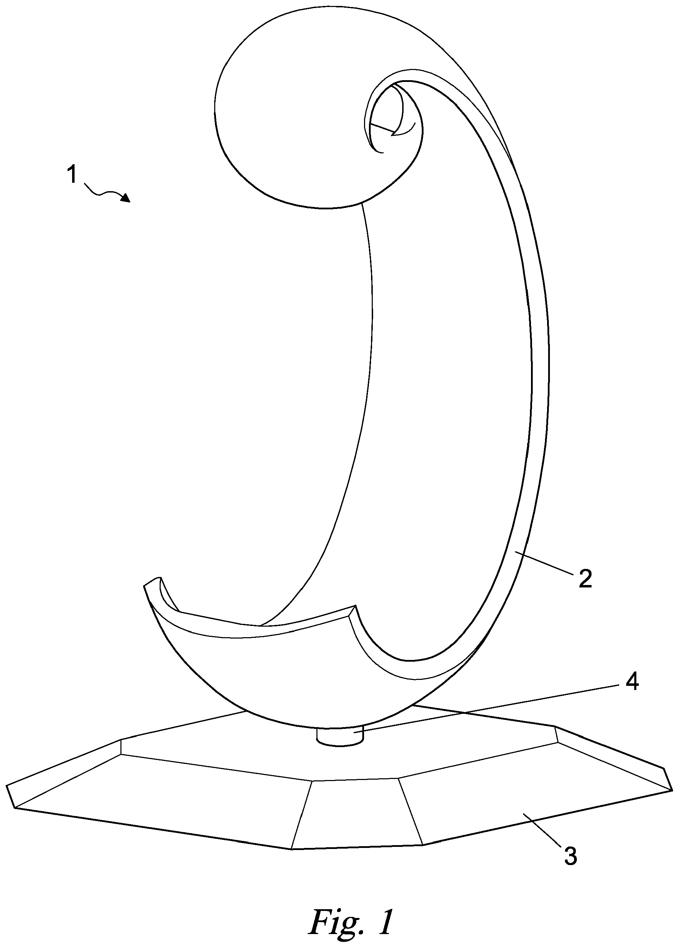

The support 2 is constituted preferably by a chair or other similar element which is adapted to allow a comfortable and ergonomic position to the user during multisensory stimulation. Preferably, the support is suitable to make the user lie in a supine position, preferably in a fetal position, inside the support 2 as shown in Figure 1.

The support 2 comprises advantageously a mat, made preferably of antibacterial material, which is adapted to cover the entire surface of the support 2 in contact with the body of the user. Such mat comprises advantageously a disposable case made of nonwoven fabric which has a border provided by an elastic.

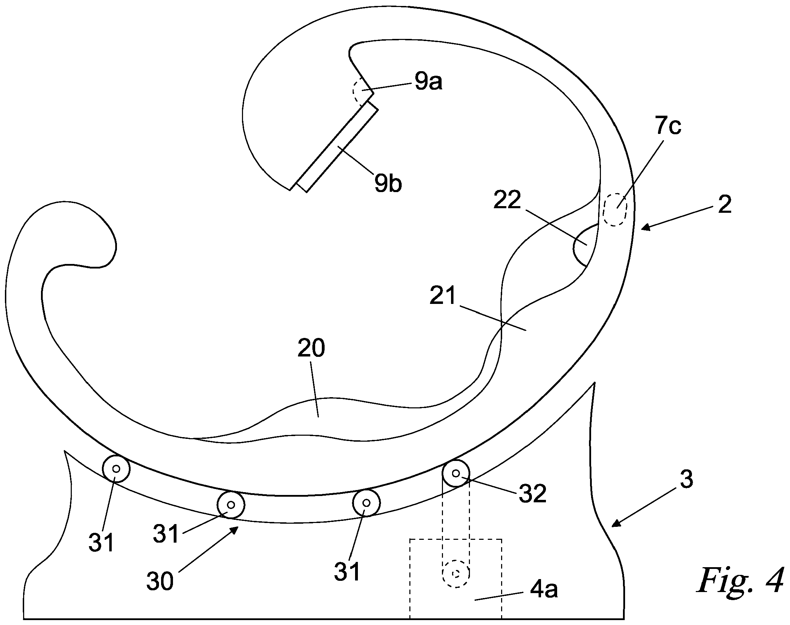

According to an embodiment of the invention shown in Figure 4, the support 2 has a supporting curvature 20 at the supporting portion in contact below the knees of the user which is adapted to facilitate leg support and correct placement of the body of the user and to avoid slippage of the user. The support 2 further comprises recessed contours 21 which are adapted to accommodate the arms of the user along its sides. The support 2 also comprises a supporting element 22 for the nape of the neck, advantageously of the type with a padded or inflatable cushion, which is adapted to support the head of the user. Advantageously means are provided suitable to vibrate said nape supporting element or, in the embodiment of said nape supporting element with inflatable cushion, means are provided adapted to inflate and deflate said cushion.

As an alternative, the support 2 can be constituted by a simple platform or other similar element that allows to user to stand erect.

The support 2 is moved by the movement mechanism 4, which comprises a motor that is adapted to move the support 2 with respect to the base structure 3 and therefore with respect to the ground or resting surface. In particular, the movement mechanism 4 turns the support 2 with respect to the base structure 3 and preferably vibrates the support 2 with respect to the base structure 3. The mechanism 4 therefore can be constituted by an eccentric motor or other similar element which is adapted to perform a tactile stimulation of the user by means of said vibration and rotation. As shown in Figure 4, the sensory stimulation device comprises means 30 for sliding engagement between the support 2 and the base structure 3. Said sliding engagement means comprise advantageously one or more rollers 31 arranged between the support 2 and the base structure 3, at least one roller 32 thereof being kinematically connected to the movement mechanism 4a. Said rollers are arranged along a circular arc, so that the actuation of said movement mechanism 4a causes an oscillation of the support 2 with respect to its base structure 3 along the circular arc defined by the arrangement of said rollers 31 and 32.

The rest of the multisensory stimulation is performed by the stimulation apparatus 5, which comprises a plurality of diffusers adapted to stimulate simultaneously the olfactory, visual and auditory senses of the user. Therefore, the apparatus 5 has speakers 7, which are adapted to stimulate the auditory senses of the user, olfactory diffusers 8, adapted to emit perfumed essences adapted to stimulate the olfactory senses of the user, and light diffusers 9 adapted to stimulate the visual senses.

The speakers 7 are preferably of the digital type, i.e., the audio is preserved and transmitted in digital form and stored on adapted digital memories, such as for example hard disks, CDs, DVDs, USB drives, SD memories and other similar tools.

Preferably, the speakers 7 are of the digital type and comprise one or more speaker enclosures 7a, which are arranged at the part of said openings of the structure 3 and are adapted to emit sound/music, and a reading system 7b, which is accommodated within the structure 3 and is adapted to read said digital memories. In particular, the speaker enclosures 7a are arranged at the support 2, while the reading system 7b is adapted to allow the insertion and removal of digital memories and to transmit the signal to the speakers 7a by means of suitable cables.

As shown in Figure 4, the speakers 7c are arranged in portions of the support 2 proximate to the ears of the user. Advantageously, the speakers 7c comprise stereophonic speaker enclosures. The speakers also comprise a jack output for the optional connection of stereophonic headphones.

The olfactory diffusers 8 are constituted by any known element which is adapted to emit a fragrance or a perfumed essence which is enclosed in adapted containers which are easily replaceable. For example, said diffusers emit perfumed essences by way of a substantially spontaneous evaporation of a liquid, the heating of a liquid or solid substance, or, as alternative, the emission of a gas stored in cans.

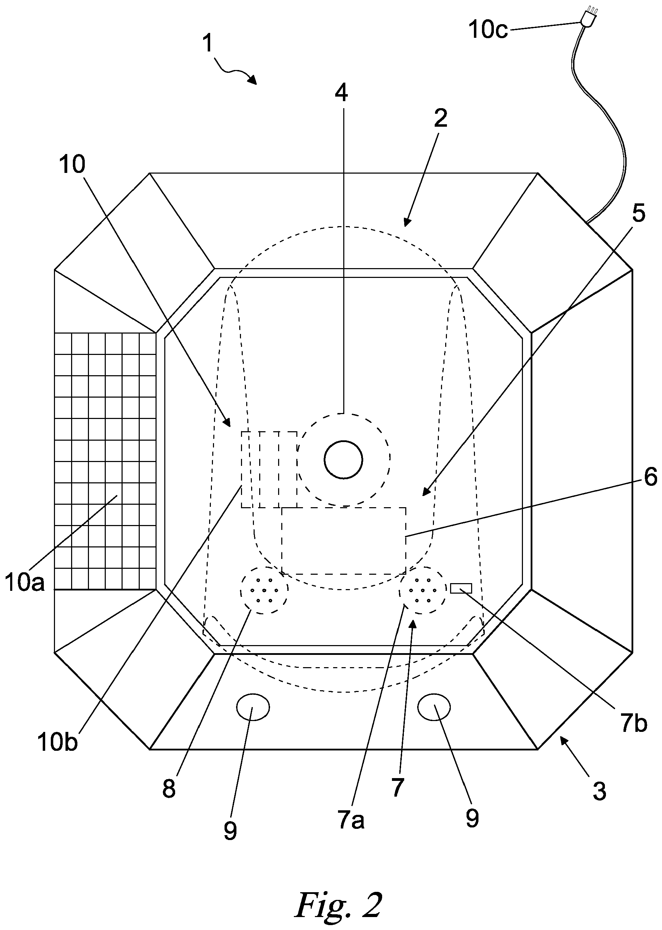

Finally, the olfactory diffusers 8 are arranged, like the speaker enclosures 7a, at openings provided in the base structure 3 and, more precisely, at the support 2, as shown in Figure 2.

The light diffusers 9 comprise one or more emission elements, such as lamps and LEDs, which emit white light or suitable combinations of colored light with different luminous intensities, conveniently accommodated in the base structure 3 and arranged at openings so as to illuminate the user when he/she is on the support 2. In detail, in order to allow correct optical stimulation, the light diffusers 9 can have mirrors or other redirection elements which are adapted to allow to focus the light on the support 2 and more precisely on the user.

In a preferred embodiment, which is not shown, the light diffusers comprise LEDs 9a which are arranged in portions of the support 2 in front of the head of the user. The light diffusers comprise a monitor 9b, advantageously of the LCD type, which is arranged in front of the face of the user and conveniently can be oriented and is adapted to display images or messages.

The device 1 comprises a power supply system 10, which is suitable to supply power to the device 1, and an activation unit 11, which is adapted to control the activation of said device.

The power supply system 10 can supply power independently, i.e., without requiring a connection to an outer electrical power supply mains, and/or in a dependent manner, i.e., by placing the device in a current flow connection with an outer electrical mains.

In detail, the power supply system 10 comprises photovoltaic panels 10a, which are adapted to convert solar energy directly into electric power, at least one battery 10b, which is adapted to store and supply electric power, and a connection element 10c, which is constituted for example by a cable provided with an electrical pin and adapted to place the device 1 in a current flow connection with an outer electrical mains. In particular, the photovoltaic panels 10a are arranged at the lateral surface of the base structure 3, i.e., in the surface that is not at the support 2 and at the ground, while the battery is accommodated within the structure 3.

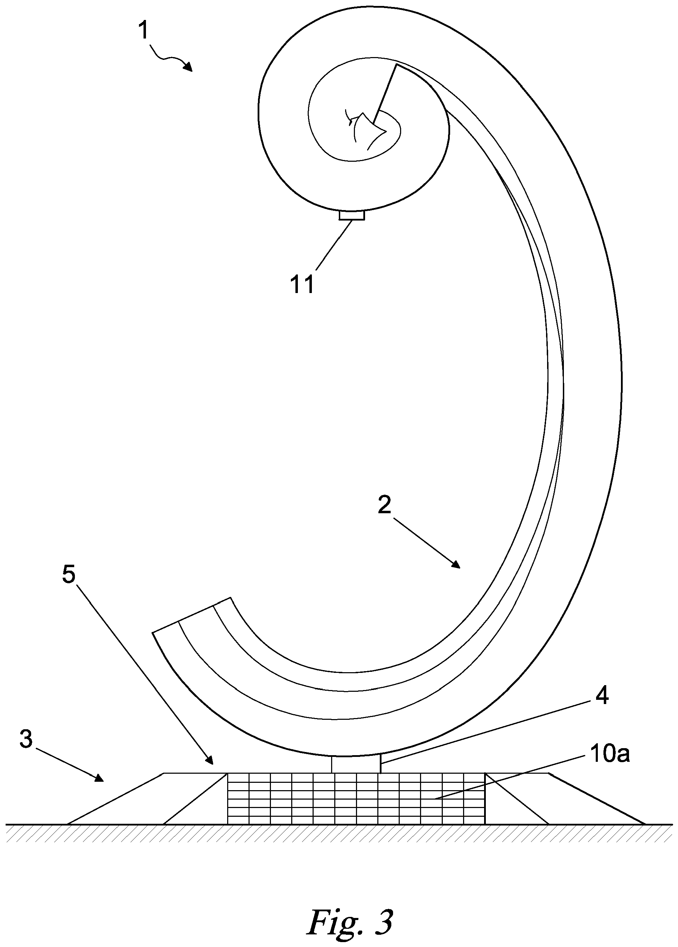

The activation unit 11, shown in Figure 3, can order, by way of the electronic card 6, the activation of the device 1 and in particular of the mechanism 4 and of the diffusers 7, 8 and 9 according to a predefined sequence or preferably simultaneously.

This activation can be manual, i.e., by means of a switch operated by the user or, as an alternative, automatic, i.e., by means of one or more sensors adapted to detect the presence of the user.

In particular, the activation unit 11 is adapted to control the activation of the device 1 automatically and therefore comprises sensors which are adapted to detect the presence of the user, for example by detecting the weight variation that occurs when the user climbs onto the support 2. Preferably, the unit 11 is arranged at the support 2.

All of the above cited functions are controlled advantageously by the electronic card 6, which controls the operation of all of the components that constitute the device.

In detail, the card 6 is capable of controlling the operation of the diffusers 7, 8 and 9 and of the mechanism 4 according to one or more preset sequences. In particular, the electronic card 6 is adapted to control the operation of the above cited components according to one or more programs which are preloaded on the card 6 or can be programmed by means of it, in order to adapt the sensory stimulation to the specific treatment that one intends to perform on the user.

In detail, the electronic card 6 can be controlled by means of a keyboard/keypad which is conveniently coupled on the base structure 3 or on the support 2.

As an alternative, the card 6 is advantageously provided with an antenna or other similar element that is adapted to allow selection of the treatment by means of a remote control or another device adapted to allow remote control of the device 1.

Operation of the device for multisensory stimulation 1, described above in a structural sense, is as follows.

Initially, the device 1 is arranged within an ordinary room, i.e., a room which is not specifically preset for multisensory therapy, and is connected to a power supply mains.

The user, by means of a keyboard or remote control, selects the multisensory stimulation program.

He/she then approaches the device 1 and places himself/herself on the support 2. This action causes the activation of the unit 11, which in turn controls, by means of the electronic card 6, the activation of the movement mechanism 4 and of the movement apparatus 5.

In detail, on the basis of the selected program and therefore of the orders given by the card 6, the mechanism 4 begins to cause the vibration, according to a preset frequency and amplitude of oscillation, of the support 2, producing a tactile stimulation on the user, while the diffusers 7, 8 and 9 begin the visual, acoustic and olfactory stimulation of the senses of the user.

The nape supporting element 22, advantageously of the type with an inflatable cushion, can be made to vibrate or inflated and deflated cyclically in order to induce a state of relaxation of the muscles of the neck of the user.

Once the multisensory stimulation program has ended, the card 6 stops the stimulation apparatus 5 and the movement mechanism 4 and therefore controls the shutdown of the device 1.

The invention allows important advantages.

The device for multisensory stimulation 1 is in fact simple to use and at low cost.

In particular, the advantageous presence of the electronic card 6 makes the device 1 easily usable without requiring a technician who controls and adjusts the several multisensory stimulation diffusers 7, 8 and 9 and the movement mechanism 4.

Furthermore, the card 6, by adjusting substantially all the operating parameters, is adapted to allow the device 1 to perform a substantially perfect treatment.

Another advantage, which is achieved with the device 1, is constituted by the possibility to perform multisensory stimulations substantially anywhere, i.e., without the need to prepare particular and expensive rooms.

A further advantage is constituted by the possibility to perform multisensory treatments that have a low cost but high quality.

The device thus conceived is susceptible of numerous modifications and variations, all of which are within the scope of the appended claims.

In practice, the materials used, as well as the shapes and dimensions, may be any according to requirements and to the state of the art.

The disclosures in Italian Utility Model No. MI2010U000367 and in Italian Patent Application No. MI2011A001082 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device (1) for multisensory stimulation, comprising a support (2) which is adapted to support the body of a user in a correct position; a base structure (3) which is adapted to support said support (2) comprising a movement mechanism (4, 4a) which is adapted to create a relative motion of said support (2) with respect to said base structure (3); a stimulation apparatus (5) which is connected to said base structure (3) and is adapted to stimulate at least part of the senses of the user; an electronic card (6) which is adapted to control said movement mechanism (4, 4a) and said stimulation apparatus (5); means (30) for sliding engagement between said support (2) and said base structure (3); said sliding engagement means comprising at least one roller (31) arranged between said support (2) and said base structure (3), said sliding engagement means also comprising at least one roller (32) kinematically connected to said movement mechanism (4a); said rollers (31, 32) being arranged along a circular arc, so that the actuation of said movement mechanism (4a) causes an oscillation of said support (2) with respect to said base structure (3) along the circular arc defined by the arrangement of said rollers (31, 32);
and **characterised in that** the support (2) comprises
- a supporting curvature (20) at the supporting portion in contact below the knees of the user which is adapted to facilitate leg support and correct placement of the body of the user and to avoid slippage of the user;
- recessed contours (21) which are adapted to accommodate the arms of the user along the sides of the body of the user, when the device is in use;
- a supporting element 22 for the nape of the neck, preferably of the type with a padded or inflatable cushion, which is adapted to support and vibrate the head of the user.

2. The device (1) according to claim **1,** wherein said relative motion of said support (2) with respect to said base structure (3) is a vibration.

3. The device (1) according to claim **1,** wherein said relative motion of said support (2) with respect to said base structure (3) is an oscillation.

4. The device (1) according to one or more of the preceding claims, wherein said stimulation apparatus (5), said movement mechanism (4, 4a) and said electronic card (6) are at least partially accommodated within said base structure (3).

5. The device (1) according to one or more of the preceding claims, wherein said stimulation apparatus (5) comprises speakers (7) which are adapted to stimulate the auditory senses of the user.

6. The device (1) according to one or more of the preceding claims, wherein said stimulation apparatus (5) comprises light diffusers (9, 9a, 9b) which are adapted to stimulate the visual senses of the user.

7. The device (1) according to one or more of the preceding claims, wherein said stimulation apparatus (5) comprises olfactory diffusers (8) which are adapted to stimulate the olfactory senses of the user.

8. The device (1) according to one or more of the preceding claims, wherein said stimulation apparatus (5) is adapted to stimulate simultaneously olfactory, visual and auditory senses of the user.

9. The device (1) according to one or more of the preceding claims, comprising an activation unit (11) which is adapted to control the activation of said device (1).

10. The device (1) according to the preceding claim, wherein said activation unit (11) comprises sensors adapted to detect the presence of the user on said support (2).

11. The device (1) according to claim 10, wherein a power supply system (10) comprises photovoltaic panels (10a).

## Patentansprüche

1. Eine Vorrichtung (1) zur multisensorischen Stimulation, umfassend eine Unterlage (2), die geeignet ist, den Körper eines Benutzers in korrekter Position zu halten, eine Basisstruktur (3), die geeignet ist, besagte Unterlage (2) zu unterstützen, umfassend einen Bewegungsmechanismus (4, 4a), der geeignet ist, besagte Unterlage (2) in Bewegung relativ zur Basisstruktur (3) zu versetzen, eine Stimulationsapparatur (5), die mit besagter Basisstruktur (3) verbunden ist und geeignet ist, zumindest einen Teil der Sinne des Benutzers zu stimulieren, eine elektronische Karte (6), die geeignet ist, den besagten Bewegungsmechanismus (4, 4a) und besagte Stimulationsapparatur (5) zu steuern, ein Mittel (30) zur gleitenden Verbindung von besagter Unterlage (2) und besagter Basisstruktur (3), wobei besagte Gleitverbindung mindestens eine Rolle (31) umfasst, die zwischen besagter Unterlage (2) und besagter Basisstruktur (3) angeordnet ist, wobei besagte Gleitverbindung mindestens auch eine Rolle (32) umfasst, die kinematisch mit besagtem Bewegungsmechanismus (4a) verbunden ist, wobei besagte Rollen (31, 32) entlang einem ringförmigen Bogen angeordnet sind, sodass die Betätigung des besagten Bewegungsmechanismus (4a) eine Wellenbewegung der besagten Unterlage (2) relativ zur besagten Basisstruktur (3) den ringförmigen Bogen entlang hervorruft, die durch die Anordnung der besagten Rollen (31, 32) festgelegt ist,
und die sich dadurch auszeichnet, dass die besagte Unterlage (2) umfasst:
- eine unterstützende Kurvenstruktur (20), an dem sich unter den Knien des Benutzers berührenden Abschnitt, die geeignet ist, die Beine des Benutzers zu unterstützen und seinen Körper in der richtigen Position zu halten und das Verrutschen des Benutzers zu verhindern;
- vertiefte Konturen (21), die geeignet sind, bei Benutzung der Vorrichtung die Arme des Benutzers neben seinem Rumpf aufzunehmen;
- ein Stützelement (22), das für den Nacken des Benutzers geeignet ist, vorzugsweise in der Art eines gepolsterten oder aufblasbaren Kissens, das geeignet ist, den Kopf des Benutzers zu unterstützen und vibrieren zu lassen.

2. Die Vorrichtung (1) nach Anspruch 1, wobei die Bewegung der besagten Unterlage (2) relativ zur besagten Basisstruktur (3) eine vibrierende Bewegung ist.

3. Die Vorrichtung (1) nach Anspruch 1, wobei die Bewegung der besagten Unterlage (2) relativ zur besagten Basisstruktur (3) eine wellenförmige Bewegung ist.

4. Die Vorrichtung (1) nach einem oder mehreren der vorstehenden Ansprüche, wobei die besagte Basisstruktur (3) die besagte Stimulationsapparatur (5), den besagten Bewegungsmechanismus (4a) und die besagte elektronische Karte (6) zumindest teilweise aufnimmt.

5. Die Vorrichtung (1) nach einem oder mehreren der vorstehenden Ansprüche, wobei die besagte Stimulationsapparatur (5) Lautsprecher (7) umfasst, die geeignet sind, den Hörsinn des Benutzers zu stimulieren.

6. Die Vorrichtung (1) nach einem oder mehreren der vorstehenden Ansprüche, wobei die besagte Stimulationsapparatur (5) lichtstreuende Elemente (9, 9a, 9b) umfasst, die geeignet sind, den Sehsinn des Benutzers zu stimulieren.

7. Die Vorrichtung (1) nach einem oder mehreren der vorstehenden Ansprüche, wobei die besagte Stimulationsapparatur (5) Duft-Diffusoren (8) umfasst, die geeignet sind, den Geruchsinn des Benutzers zu stimulieren.

8. Die Vorrichtung (1) nach einem oder mehreren der vorstehenden Ansprüche, wobei die besagte Stimulationsapparatur (5) geeignet ist, Geruch-, Seh- und Hörsinn des Benutzers gleichzeitig zu stimulieren.

9. Die Vorrichtung (1) nach einem oder mehreren der vorstehenden Ansprüche, mit einer Aktivierungseinheit (11), die geeignet ist, die Aktivierung der besagten Vorrichtung (1) zu steuern.

10. Die Vorrichtung (1) nach einem oder mehreren der vorstehenden Ansprüche, wobei besagte Aktivierungseinheit (11) Sensoren umfasst, die die Anwesenheit des Benutzers auf der besagten Unterlage (2) feststellen.

11. Die Vorrichtung (1) nach Anspruch 10, wobei eine Stromversorgung (10) photovoltaische Elemente (10a) umfasst.

## Revendications

1. Dispositif (1) de stimulation multisensorielle, comprenant un support (2) qui est conçu pour supporter le corps d'un utilisateur dans une position correcte ; une structure de base (3) qui est conçue pour supporter ledit support (2) comprenant un mécanisme de mouvement (4, 4a) qui est conçu pour créer un mouvement relatif dudit support (2) par rapport à ladite structure de base (3) ; un appareil de stimulation (5) qui est connecté à ladite structure de base (3) et est conçu pour stimuler au moins une partie des sens de l'utilisateur ; une carte électronique (6) qui est conçue pour commander ledit mécanisme de mouvement (4, 4a) et ledit appareil de stimulation (5) ; un moyen (30) assurant un engagement coulissant entre ledit support (2) et ladite structure de base (3) ; ledit engagement coulissant comprenant au moins un galet (31) agencé entre ledit support (2) et ladite structure de base (3), ledit moyen d'engagement coulissant comprenant également au moins un galet (32) raccordé cinématiquement au dit mécanisme de mouvement (4a) ; lesdits galets (31, 32) étant agencés sur un arc circulaire, de telle manière que l'actionnement dudit mécanisme de mouvement (4a) provoque une oscillation dudit support (2) par rapport à ladite structure de base (3) le long de l'arc circulaire défini par l'agencement desdits galets (31, 32) ;
et **caractérisé en ce que** le support (2) comprend
- une courbure de support (20) au niveau de la partie de support en contact sous les genoux de l'utilisateur, qui est conçue pour faciliter le support de la jambe et corriger la position du corps de l'utilisateur et éviter le glissement de l'utilisateur ;
- des contours en creux (21) qui sont conçus pour recevoir les bras de l'utilisateur le long des côtés du corps de l'utilisateur, lorsque le dispositif est en cours d'utilisation ;
- un élément de support (22) pour la nuque, au niveau du cou, de préférence du type muni d'un coussin matelassé ou gonflable, qui est conçu pour supporter et faire vibrer la tête de l'utilisateur.

2. Dispositif (1) selon la revendication 1, dans lequel ledit mouvement relatif dudit support (2) par rapport à ladite structure de base (3) est une vibration.

3. Dispositif (1) selon la revendication 1, dans lequel ledit mouvement relatif dudit support (2) par rapport à ladite structure de base (3) est une oscillation.

4. Dispositif (1) selon l'une ou plusieurs des revendications précédentes, dans lequel ledit appareil de stimulation (5), ledit mécanisme de mouvement (4, 4a) et ladite carte électronique (6) sont au moins en partie logés à l'intérieur de ladite structure de base (3).

5. Dispositif (1) selon l'une ou plusieurs des revendications précédentes, dans lequel ledit appareil de stimulation (5) comprend des haut-parleurs (7) qui sont conçus pour stimuler le sens de l'ouïe de l'utilisateur.

6. Dispositif (1) selon l'une ou plusieurs des revendications précédentes, dans lequel ledit appareil de stimulation (5) comprend des diffuseurs de lumière (9, 9a, 9b) qui sont conçus pour stimuler le sens de la vue de l'utilisateur.

7. Dispositif (1) selon l'une ou plusieurs des revendications précédentes, dans lequel ledit appareil de stimulation (5) comprend des diffuseurs olfactifs (8) qui sont conçus pour stimuler le sens de l'odorat de l'utilisateur.

8. Dispositif (1) selon l'une ou plusieurs des revendications précédentes, dans lequel ledit appareil de stimulation (5) est conçu pour stimuler simultanément les sens de l'odorat, de la vue et de l'ouïe de l'utilisateur.

9. Dispositif (1) selon l'une ou plusieurs des revendications précédentes, comprenant un système d'actionnement qui est conçu pour commander l'actionnement dudit dispositif (1).

10. Dispositif (1) selon la revendication précédente, dans lequel ledit système d'actionnement (11) comprend des capteurs conçus pour détecter la présence de l'utilisateur sur ledit support (2).

11. Dispositif (1) selon la revendication 10, dans lequel un système d'alimentation électrique (10) comprend des panneaux photovoltaïques (10a).
